# EUROPEAN PATENT SPECIFICATION

(11) **EP 0 269 086 B1**
(45) Date of publication and mention of the grant of the patent: **17.03.1993**
(21) Application number: 87117395.1
(22) Date of filing: 25.11.1987
(51) Int. Cl.: C12M 1/40, C12M 3/02

(54) **Cell culture apparatus**
Zellkulturapparat
Appareil pour la culture de cellules

(30) Priority: 26.11.1986 JP 281192/86
(43) Date of publication of application: 01.06.1988
(73) Proprietor: RIKAGAKU KENKYUSHO, Wako-shi Saitama-ken (JP); SHINKO PANTEC CO., LTD., Kobe-shi, Hyogo-ken (JP)
(72) Inventor: Endo, Isao, Kokubunji-shi Tokyo (JP); Nagamune, Teruyuki, Chuo Kamifukuoka-shi Saitama-ken (JP); Kobayashi, Tetsuo, Kita-ku Kobe-shi Hyogo-ken (JP)
(74) Representative: VOSSIUS & PARTNER

(56) References cited:
- EP-A- 0 197 299
- BIOTECH' 83, PROCEEDINGS OF THE INTERNATIONAL CONFERENCE ON THE COMMUNICAL APPLICATIONS OF BIOTECHNOLOGY, London, 4th - 6th May 1983, pages 597-610, Northwood, GB; G. ODA et al.: "Continuous alcohol fermentation technologies using immobilized yeast cells"

## Description

This invention relates to a cell culture apparatus in which microorganisms, particularly fungi such as molds, actinomycetes, animal cells, or plant cells are attached to carriers of porous material which are fluidized in liquid medium by air flow and grow to continually produce useful substances such as antibiotics, enzymes, proteins, polysaccharides, physiologically active substances, or animal and plant hormones.

It is well known that submerged culture methods have been used in cultures of molds or actinomycetes, and primary metabolites, such as glycerin or alcohol and secondary metabolites, such as penicillin or streptomycin have been produced on an industrial scale by means of an air flow agitation tank.

A novel cell culture method and an apparatus therefor was disclosed in unexamined Japanese Patent Publication No. 60(1985)-21487 and Japanese Utility Model Application No. 60-23142, respectively. In the cell culture method,the cells to be proliferated are attached to a porous material suspended in liquid medium and the cell culture is carried out in much higher efficiency than in the prior art since the cells grow in porous material instead of liquid medium. In the cell culture apparatus, cell growth and separation of cells from the liquid medium including metabolites can be carried out in a single apparatus. The above proposed method and apparatus attracted attention in the field because the production efficiency of metabolites such as penicillin is extremely high.

For instance, the prior art cell culture methods employ submerged cell culture methods which use an air flow agitation tank. In these submerged cell culture methods, liquid medium is periodically or continuously supplied and taken out to allow continuous culture. However, these culturing operations cause problems, such as the flow of proliferated cells, the necessity of large culture apparatus,and the necessity of large agitation power, so that it is difficult to continuously produce metabolites. Furthermore, when the metabolites are not secreted into the culture broth, it is very difficult to continuously and selectively take out cells in which large amounts of the metabolite are accumulated. Therefore, a batch operation is often used in this case.

The above proposed method and apparatus differ from the prior art in that the cells to be proliferated are attached to porous materials,such as formed synthetic high polymer, e.g. urethane foam; natural sponge; natural fibers, such as cotton; or to a porous material made of synthetic fibers. In this method the culture of cells is carried out in extremely high efficiency and, furthermore, the liquid medium including the metabolites can easily be separated from the cells. However, even in the above proposed apparatus the cell culture cannot be carried out continuously.

Therefore, it is an object of this invention to provide an improved cell culture apparatus which allows the continuous production of metabolites.

The object is achieved by providing a cell culture apparatus comprising a fluidized bed culture body in which cell attached porous material is fluidized by air flow and cells are proliferated, and
a pre-culture tank for supplying medium to said fluidized bed culture body corresponding to the amount of liquid medium which is taken out of said fluidized bed culture body.

This invention is different from the prior art cell cultures, especially the submerged culture in which cells are proliferated in a batch-wise or continuous operation using an air flow agitator. In this invention, liquid medium including the metabolite is taken out singly or along with some pieces of cell attached porous material and newly adjusted medium is supplied singly or along with porous material to the culture tank body so that the metabolite is continuously produced.

More specifically, in this invention, liquid medium and porous material are firstly supplied to the air flow fluidized bed culture tank body. The diameter of the porous material is preferably below one tenth of a typical diameter of the fluidized bed culture tank body or below 10 mm and the volume of the porous material is preferably 0.1 to 0.7 liter per one liter of liquid medium. After sterilization of the culture body, the cells are inoculated into the culture body and the air flow fluidized culture is started. The proliferation of the cells is enhanced by a biological film formed on the surface of the porous material. By detecting the time when the concentration of the metabolite in the liquid medium reaches a predetermined concentration , the time when the concentration of the substrate decreases to a predetermined concentration, or the cell growth state, or the amount of the produced metabolite, by measuring the parameters such as pH or DO of the liquid medium, the amount of the liquid medium to be taken out and the time when the takeout operation starts are determined. Then the liquid medium is taken out, being separated from the cell attached porous material or along with some pieces of the cell attached porous material from a drain pipe through a valve.

Through a supply pipe connected with the pre-culture tank through a valve, newly adjusted medium is supplied singly or along with the porous material to the fluidized bed culture tank, and air flow fluidization is continuously or sequentially carried out. As a result, the cells attached to the porous material which remains in the fluidized bed culture tank allows the continuous production of metabolites.

A perforated valve having perforations smaller than the size of the porous material may be provided within the drain pipe so that the liquid material is separated from the cell attached porous material .

According to this invention, the following advantages can be obtained:
1. It is easy to carry out the takeout and supply operation on a condition adequate for the growth properties of each kind of cells. Specifically, it becomes possible to carry out a continuous or sequential cell culture which has been difficult in the prior art using an air flow agitator and recovering the desired metabolite separately and continuously. Therefore, this invention has very great effect on the industrial production of metabolites.
2. Since this invention allows the continuous or sequential culture of cells which have been attached to porous material , it becomes possible to delete or shorten e.g. sterilization operations, or cell inoculation operations which are to be carried out for each batch of the prior art batch-wise culture methods. Production efficiency increases as a whole. In other words, the productivity of a useful substance such as antibiotics (streptomycin, cephamycin, oxytetracycline, erthoromycin, kanamycin, etc.), enzymes(amylase, protease, pectinase, cellulase, lipase, glucoamylase, glucoisomerase, etc.), proteins(single cell protein, etc.), polysaccharides (arabinose, mannose, rhamnose, dextran, etc.), physiological active substances(vitamin B₁₂, vitamin₂, vitamin C, etc.), or animal or plant hormones (auxin, cytokinin, gibberellin, steroid hormone, insulin, lymphokine, etc.) is enhanced. In summary, this invention can be referred to as a separator type bioreactor by which mass production can be continuously carried out and the desired product can be recovered separately.

The Figures show:
Fig. 1 is a schematic diagram of a preferred embodiment of the cell culture apparatus;
Fig. 2 is a schematic diagram of another preferred embodiment of this invention, which is used especially when liquid medium has to be separated from porous material;
Fig. 3 is a schematic diagram of still another preferred embodiment of this invention, which is used when liquid medium is continuously taken out and supplied;
Fig. 4 is a sectional view of a valve which can pass liquid medium only through a drain or drawn pipe;
Fig. 5 is a graph showing time dependent properties of a Penicillium chrysogenum JCM 2056 strain under culture conditions where only liquid medium is taken out and newly adjusted medium is supplied;
Fig.6 is a graph showing time dependent properties of the JCM 2056 strain under culture conditions where liquid medium and porous material are taken out, and newly adjusted medium and new porous material are supplied.

The Examples illustrate the invention:

### Example

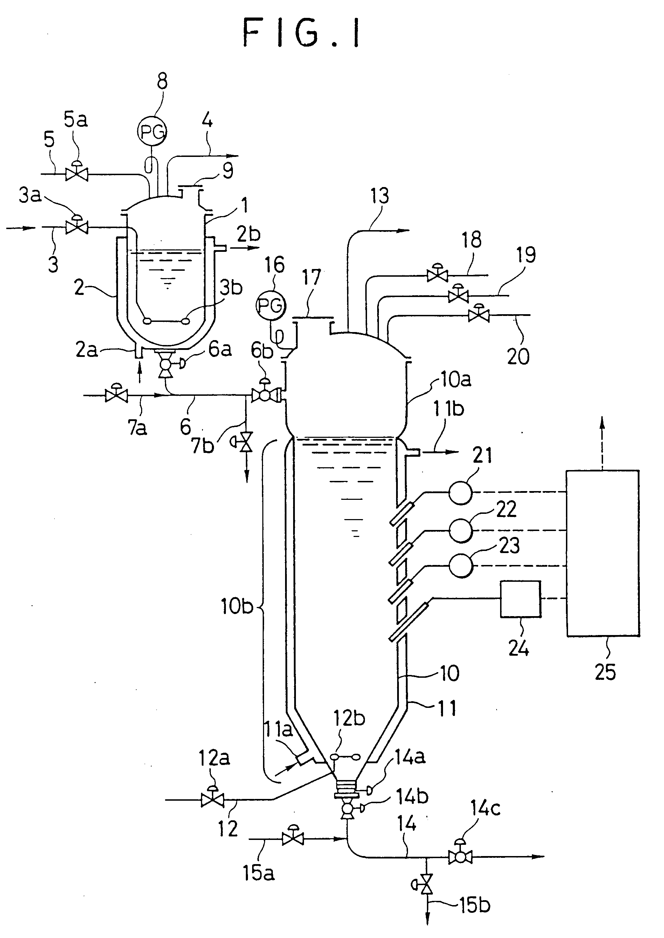

Fig.1 shows a preferred cell culture apparatus according to this invention.

The cell culture apparatus according to this invention comprises a pre-culture tank 1 for supplying liquid medium and porous material or liquid medium only and a fluidized bed culture tank body 10. A supply pipe 6 having automatic control valves 6a, 6b connects the pre-culture tank 1 and the fluidized bed cell culture tank body 10 and transfers medium or porous material. A drain pipe 14 having automatic control valves 14a, 14b is provided at the bottom of the culture tank body 10 and drains liquid medium or porous material.

The automatic control valve 14a is provided toward the fluidized bed culture tank body 10 with respect to the automatic control valve 14b and has perforations smaller than the size of the porous material.

Supply pipes 7a, 15a for supplying sterilized and pressurized air or vapor for sterilization and drain pipes 7b, 15b for draining liquid medium or porous material are also provided.

The upper part of the fluidized bed culture tank body 10 forms a freeboard, and the lower part forms a porous material fluidized bed culture portion 10b.

Air flow pipes 3, 12 with automatic control valve 3a or 12a, air diffusers 3b, 12b, exhaust pipes 4, 13, porous material introduced ports 9, 17 are provided with the pre-culture tank 1 and the culture tank body 10,respectively. Constant temperature jackets 2, 11 are further provided around the tank 1and the tank body 11, respectively, and temperature controlled water flows through the jackets 2, 11 from temperature controlled lower inlets 2a, 11a to temperature controlled upper outlets 2b, 11b so that culture is carried out under a predetermined culture temperature.

An inoculation nozzle 18, an acid/alkali injection nozzle 19, and anti-foaming agent injection nozzle 20 are provided with the culture tank body 10. A medium injection pipe 5 having an automatic injection valve 5a is provided with the pre-culture tank 1.

At the beginning of culture, porous material and medium of a predetermined amount for starting culture are taken into the fluidized bed culture tank body 10. After sterilization of those, cells are inoculated from the inoculation nozzle 18. Air, which may be enriched with oxygen, is introduced at a predetermined rate through the air flow pipe 12 and the air-diffuser 12b which is disposed at the bottom of the culture tank body 10 and has many openings smaller than the size of the porous material. And then fluidized cell culture starts.

The culture condition is detected by a pH sensor 21, a DO sensor (Dissolved Oxygen concentration meter) 22, a temperature sensor 23, and an automatic analyzer 24 ( which causes regular sampling by means of a timer controller, and then automatically measures substrate and metabolite concentration by using a liquid chromatography and also may measure cell concentration by using a turbidity meter ) . The detected values are monitored and recorded by a control device 25 including a computer. The control device compares a preprogramed changing pattern of the concentration of the metabolite or substrate or a preprogramed changing patternof the pH or DO value as indirect observations under culture with the detected values in order to estimate the time when the concentration of the metabolite in the liquid medium reaches a predetermined concentration or the time when the concentration of the substrate reaches a predetermined concentration. If a controller without a computer, e.g. a sequence controller is used, the time when the concentration reaches a predetermined value is directly detected. As a result of the estimation, the time to start taking out and supplying liquid medium and porous material is determined. The amount of proliferated cells may be estimated from the amount of the produced metabolite and the amount of consumed substrate, further the amount of the cell attached porous material and liquid medium to be taken out is determined.

By actuating the automatic control valves, the porous material and the liquid medium of the determined amount are taken out through the drain pipe 14 disposed at the bottom of the fluidized bed culture tank body 10. New porous material and newly adjusted medium are supplied through the supply pipe 6 to the fluidized bed culture tank body 10 to carry out the fluidized culture continuously. If the sequencer controller is used, the time to change the porous material and the liquid medium is predetermined by a timer controller or determined by an indicated value of a level indicator, so that control for supplying porous material and liquid medium of the predetermined are carried out.

The above described operations may vary depending on the kinds of cells, but in all cases, some pieces of cells attached porous material are left in the culture tank body 10.

The cells, which live on the surface layer of the porous material left in the tank body 10, grow using the supplied new porous material and newly adjusted medium. Therefore, sterilization operations, inoculation operations and cell proliferation time, which have to be carried out for each batch process employed in submerged culture methods using an air flow agitator, can be deleted or shortened.

By repeatedly carrying out the culture operations as described, the cells can continuously be propagated by use of one fluidized bed culture tank. This embodiment is especially suitable in case a desired metabolite is produced by the secondary metabolism process as in molds. In this embodiment, the desired product is continuously produced. This is the difference to the prior art batch-wise culture methods.

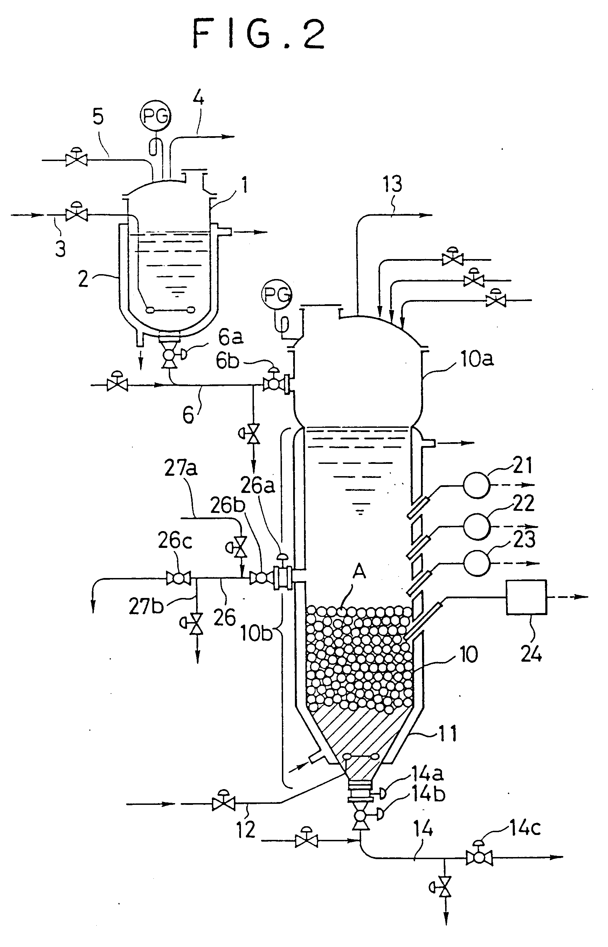

Fig.2 shows another preferred embodiment of this invention, which is used especially when liquid medium has to be separated from porous material and taken out.

A drawn pipe 26 with an automatic control porous valve 26a and automatic control drawn valves 26b and 26c are provided at the side of porous material fluidized culture portion 10a of the culture tank body 10 above a static porous material layer A which is formed in the porous material fluidized culture portion 10b while air flow stops. In this embodiment, the liquid medium can drain without friction of porous material and faster than in the case when only liquid medium drains from the drain pipe 14, which is disposed at the bottom of the culture tank body 10, through the automatic control valve.

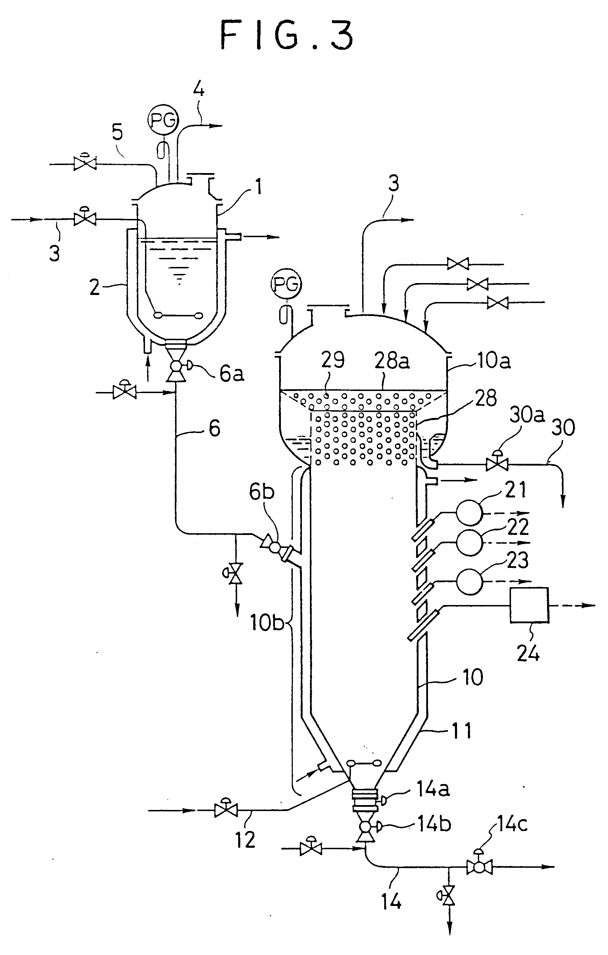

Fig.3 shows still another preferredembodiment of this invention in which the takeoutoperation and supply operation of liquid medium are continuously carried out.

A perforated cylinder 28 having many perforations 28a smaller than the size of the porous material is provided within the upper freeboard 10a of the fluidized bed culture tank body 10 and is connected to the porous material fluidized culture portion 10b. A perforated conical cylinder 29 is connected to the top of the perforated cylinder 28 and is in contact with the inner side of the freeboard 10a. A drain pipe 30 with an automatic control valve 30a is connected with the lower portion of the freeboard 10a. The supply pipe 6 from the bottom of the pre-culture tank 1 is connected to the side of the porous material fluidized culture portion 10b below the perforated cylinder 28. The liquid medium of the same amount as that which is continuously supplied from the pre-culture tank 1 is separated from the porous material by perforations 28a of the perforated cylinder 28 and the perforated conical cylinder 29 and is drained through the drain pipe 30.

The cell culture apparatus as constructed above is effective especially when large amounts of cells should be proliferated or extracellular metabolites should be continuously separated and recovered.

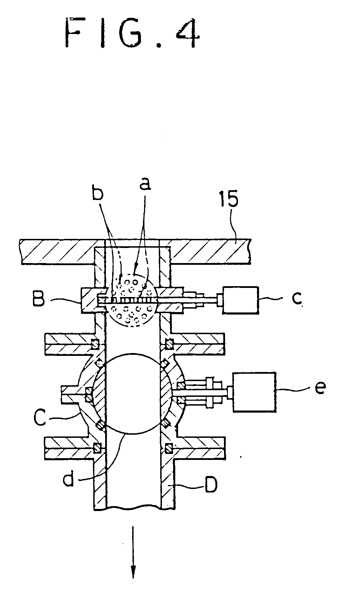

Fig.4 shows a sectional view of a drain or drawn pipe which may be used in this invention.

An automatic control perforated valve body B, in which a perforated valve a having many perforations b is installed, is provided within the drain or drawn pipe D toward the culture tank body 15. An automatic control ball valve body C, in which a ball valve d is installed, is also provided within the drain or drawn pipe D on the downstream side with respect to the automatic control perforated valve body B. When only culture liquid is drained from the culture tank body 15, the perforated valve a is rotated by a valve drive motor c to be closed, and the ball valve d is rotated by a valve drive motor e to be opened. As a result of this, pieces of the porous material are intercepted by the perforated valve a and only liquid medium drains.

When the porous material and liquid medium are both drained, the perforated valve a is also opened and the porous material is drained along with the liquid medium.

It is needless to say that the automatic perforated valve body B and/or the automatic control ball valve C may be a butterfly valve or a flush valve, and further a pressurized air driven piston may be used instead of the valve drive motor.

Hereinbelow, experimental results using a cell culture apparatus according to this invention will be shown.

### EXAMPLE 1: Method in which only liquid medium is changed.

Pieces of cubic porous material about 5 mm in side length of urethane foam 160g in total weight ( about 3000 cm³) and 8 liter of liquid medium consisting of 40g lactose, 20g corn steep liquor, 3g NaNO₃, 0.5g KH₂PO₄, 0.25g MgSO₄·7H₂O with one liter distilled water were put into a glass cylinder with a diameter of 17 cm as a fluidized bed type culture tank. Next, the culture tank including the porous material and the liquid medium was sterilized. After this sterilization, Penicillium chrysogenum JCM2056 ( Q176 ) was inoculated as penicillin production strain. The culture temperature was kept at 24°C by a temperature controlled water flow through the jacket. Air flow fluidized culture was started by an air flow at the rate of 30 l/min. The pH of the liquid medium was 4 at the beginning of culture. The pH value was monitored with a pH sensor and controlled so as not to exceed 6.

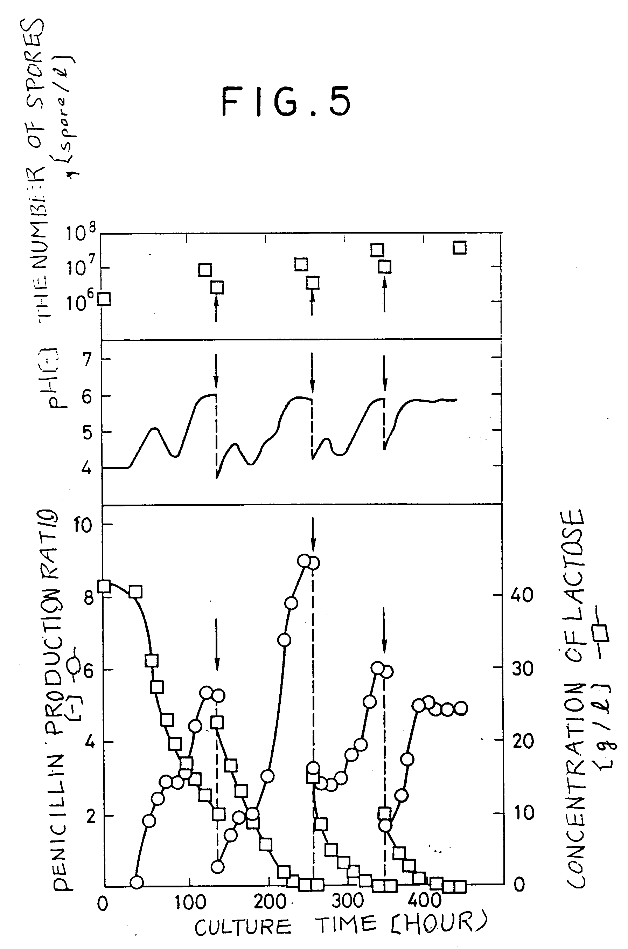

When the concentration of the produced penicilin reached the maximum, that is, after 140 hours from the beginning of culture, 6 liter of the liquid medium was singly drawn out of the fluidized bed culture tank. 6 liter of the above medium with distilled water was supplied from the pre-culture tank to the fluidized bed culture tank. The initial concentration of lactose after this change of liquid medium was 25g/liter. Then the air flow fluidized culture was carried out again. After an additional 100 hours, only the liquid medium was drawn and supplied, in a similar way as in the first operation, so that the initial concentration of lactose after this change became 15g/liter. Then, the air flow fluidized culture was restarted. After a further 90 hours, only liquid medium was taken out and supplied again so that the initial concentration of lactose after this change became 10g/litre, and then the air flow fluidized culture was restarted again. The results of this experiment are shown in Fig. 5.

In Fig.5, the amount of produced penicillin is shown in relative values,that is, in a penicillin production ratio ( - ) where a unit is selected as the maximum amount of produced penicillin obtained on the seventh day from the beginning of culture on condition that 100 ml of the above liquid medium without urethane foam was put into a 300 ml conical flask and culture was carried out by shaking culture at 200rpm rotational shaking speed at 24°C. In Fig.5, time dependent properties of the pH value and the number of spores are also shown.

After the changes of liquid medium, in each case, the ability to produce penicillin was maintained and penicillin tended to be produced in proportion to the initial concentration of lactose after the changes of medium. In the initial culture process just after the inoculation of the cells, there was a time duration while penicillin was not produced, however, production of penicillin was quickly restarted just after the exchanges of liquid medium.

During culture, the concentration of penicillin increased in proportion to the pH value, and tended to reach the maximum concentration 10 to 20 hours after pH reached 6. From the experimental results, it is found that pH indirectly showed the concentration of the metabolite such as penicillin and may be used as an index for detecting the time to start drawing and supplying medium.

In this experiment, since the porous material was not changed, the amount of the cells which grew on the surface layer of the porous material gradually increased, the number of the spores in the liquid medium also increased and mycelium grew in the liquid medium. As a result, the liquid medium became turbid.

### EXAMPLE 2 : Method in which liquid medium and porous material are both changed.

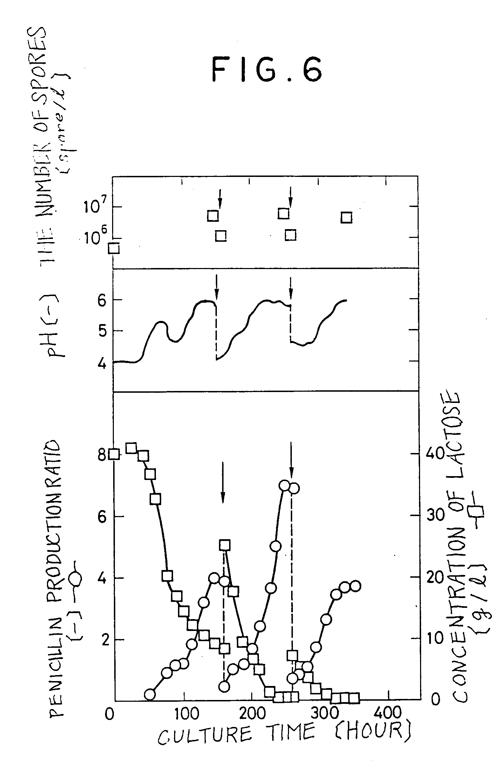

The same initial culture condition as EXAMPLE 1 was set. After 150 hours from the beginning of culture, 6.2 liter of the liquid medium and 30 % of the porous material to the initially supplied amount were taken out and new porous material and the newly adjusted medium of the same amount each as that taken out were supplied, so that the initial concentration of lactose was 25 g/liter just after this change. Then air flow fluidized culture was started again. When 100 hours lapsed, in a similar manner as the first operation, 6.5 liter of the liquid medium and 50% of the porous material to the initially supplied amount was changed so that the initial concentration of lactose just after this change was 7.5 g/liter. The air flow culture was restarted. The results of this experinent are shown in Fig. 6.

The ability to produce penicillin was maintained after the changes of the liquid medium and the porous material as in EXAMPLE 1, and the concentration of penicillin increased just after the changes.

When part of the pieces of mycelium attached porous material were taken out, and new porous material was supplied, it was found that mycelium grew on the surface layer of the porous material, as a result, the number of spores in the liquid medium was kept constant, and the growth of mycelium in the liquid medium was weaker than in EXAMPLE 1.

As descrived above, by utilizing mycelium which is in initial culture process, culture can be carried out continuously in one culture tank. It is also found when the desired metabolite to be produced is a secondary metabolite which is produced in a period while the growth of mycelium degenerates or on a low substrate concentration, the desired metabolite can be continuously produced effectively by use of a low initial substrate concentration which means a decrease of the sugar source to be consumed.

As this invention may be embodied in several forms without departing from the spirit of the essential characteristics thereof, the above embodiments are therefore illustrative and not restrictive, since the scope of the invention is defined by the appended claims rather than by the description preceding them, and all changes that fall within the bounds of the claims, or the equivalence of such bounds, are therefore intended to be embraced by the claims.

## Claims

1. A cell culture apparatus comprising:
a fluidized bed culture tank body 10 in which cell attached porous material is fluidized by air flow, and
a pre-culture tank 1 for supplying medium to said fluidized bed culture tank body corresponding to the amount of the liquid medium which is taken out of said fluidized bed culture tank body 10.

2. The cell culture apparatus of claim 1, in which said liquid medium is singly taken out of said culture tank body 10.

3. The cell culture apparatus of claim 1 or 2, in which said liquid medium is taken out of said culture tank body 10 along with said porous material, and said medium is supplied along with the porous material from said pre-culture tank 1.

## Patentansprüche

1. Zellkulturapparat, umfassend:
ein Wirbelschicht-Kulturgefäß (10), in dem an den Zellen haftendes poröses Material durch Luftströmung verwirbelt wird, und
ein Vorkulturgefäß (1) für die Zufuhr von Medium in das Wirbelschicht-Kulturgefäß entsprechend der Menge des flüssigen Mediums, das aus dem Wirbelschicht-Kulturgefäß (10) entfernt wird.

2. Zellkulturapparat nach Anspruch 1, wobei nur das flüssige Medium aus dem Kulturgefäß (10) entfernt wird.

3. Zellkulturapparat nach Anspruch 1 oder 2, wobei das flüssige Medium zusammen mit dem porösen Material aus dem Kulturgefäß (10) entfernt wird und das Medium zusammen mit dem porösen Material aus dem Vorkulturgefäß (1) zugeführt wird.

## Revendications

1. Appareil de culture cellulaire comprenant :
un corps de réservoir de culture à lit fluidisé (10) dans lequel un matériau poreux auquel sont fixées des cellules est fluidisé par écoulement d'air et
un réservoir de pré-culture (1) destiné à fournir du milieu audit corps de réservoir de culture à lit fluidisé, en une quantité correspondant à la quantité de milieu liquide retirée dudit corps de réservoir de culture à lit fluidisé (10).

2. Appareil de culture cellulaire selon la revendication 1, dans lequel ledit milieu liquide seul est retiré dudit corps de réservoir de culture (10).

3. Appareil de culture cellulaire selon la revendication 1 ou 2, dans lequel ledit milieu liquide est retiré dudit corps de réservoir de culture (10) avec ledit matériau poreux et ledit milieu est introduit avec le matériau poreux à partir dudit réservoir de pré-culture (1).
